# EUROPEAN PATENT APPLICATION

(11) **EP 4 085 873 A1**
(43) Date of publication of application: **09.11.2022**
(21) Application number: 21172850.6
(22) Date of filing: 07.05.2021
(51) Int. Cl.: A61F 2/28, A61B 17/80, A61F 2/46

(54) **DEVICE, TOOL AND SYSTEM FOR MINIMALLY INVASIVE ORBITAL FLOOR FRACTURE IMPLANTATION**

(71) Applicant: Ostbayerische Technische Hochschule Regensburg, 93049 Regensburg (DE); Universitätsklinikum Regensburg, 93053 Regensburg (DE); Universität Regensburg, 93053 Regensburg (DE)
(72) Inventor: Kühnel, Thomas, 93053 Regensburg (DE); Burger, Moritz, 93053 Regensburg (DE); Schratzenstaller, Thomas, 93053 Regensburg (DE)
(74) Representative: Lucke, Andreas

(57) **Abstract**

The invention refers to an implant device (10) for treating an orbital floor fracture. The implant device (10) is foldable in a first direction between a folded state and an unfolded state and is plastically extendible in a second direction from an unextended state to an extended state. In the extended state, the implant device (10) has an extension in the second direction that is longer than a length in the unextended state and equal to or greater than an aperture distance of a human maxillary sinus in an anterior-posterior direction. The invention further refers to a related implantation tool and a related implantation system for treating orbital blow fractures and to a corresponding implantation method.

## Description

### FIELD OF THE INVENTION

The present invention is in the field of medical technology related to treatment of orbital floor fractures. In particular, the invention refers to an implant device, an implantation tool and an implantation system for treating orbital floor fractures and to a corresponding implantation method.

### BACKGROUND OF THE INVENTION

A violent impact in the eye region of a subject can result in traumatic damage of the bone structure surrounding the eye orbit and forming the walls thereof. This bone structure comprises parts of the frontal, ethmoid, lacrimal, maxilla, zygoma, sphenoid, and palatine bones. The bones of the roof and the lateral walls of the eye orbit are relatively robust, while the comparatively thin bone of the orbital floor, which separates the eye orbit from an underlying opening called the maxillary sinus, has no structural support. Therefore, a fracture of the orbital floor is a typical injury. About 4% to 16% of all bone fractures in the facial region of the body are orbital floor fractures, mostly so-called orbital blowout fractures, typically resulting from traffic or sport accidents or from violent attacks. About 15.000 cases of orbital floor fracture are treated yearly only in the Federal Republic of Germany.

An orbital floor fracture can cause soft tissues such as fat or optic muscles to prolapse through the fractured bone into the maxillary sinus and thereby lead to a number of symptoms including orbital pain, eye displacement, in particular posterior eye displacement into the eye orbit (enophthalmos), limitation of eye movement, seeing-double (diplopia). Therefore, corrective surgery is often required to reconstruct the fractured bone in order to eliminate such symptoms.

The reconstruction of the orbital floor is usually performed with an implant through an incision formed in the eye itself (transconjunctival incision) or via infraorbital or lower eyelid incision (subciliary incision). Such incision approaches typically require cranially displacing the eyeball for positioning the implant on the orbital floor and fixating it to surrounding bone tissue. An implant plate usable for such purposes is known from US 5,743,913, which comprises a plurality of holes adapted to receive fasteners for fixating the implant plate to the surrounding bone structure. The type of surgical access is usually chosen in consideration of the location and the size of the fracture and of the surgeon's preference and experience.

Major complications related to such implantation techniques are the risk of damage to the mobility of the lower eyelid, in particular as a consequence of the incision cut formation or healing, damage to the lacrimal apparatus, and the risk of damage to the optic nerve, in particular as a consequence of the required displacement of the eyeball.

Further disadvantages of the aforementioned transconjunctival and subciliary approaches are the difficult access for the surgeon to the damaged area to be implanted, in particular in cases in which the orbital floor fracture is located in the most posterior part of the orbital floor, and the difficult handling of fatty tissues hanging down through the fractured bone from the cranial side of the orbital floor.

Therefore, there is room for technical improvement in the field of surgical treatment and implantation of orbital floor fractures.

### SUMMARY OF THE INVENTION

The problem underlying the present invention is to provide less invasive treatment possibilities for orbital floor fractures having a reduced risk of secondary effects and increased surgical simplicity. This problem is solved by an implant device according to claim 1, by an implantation tool according to claim 11 and by an implantation system according to claim 15. Preferred embodiments of the invention are defined in the appended dependent claims. The present disclosure further refers to a related method of providing an implant according to the invention at a site of an orbital floor fracture of a subject.

A first aspect of the invention refers to an implant device for treating an orbital floor fracture, in particular an orbital blowout fracture.

The implant device of the invention is foldable in a first direction between a folded state and an unfolded state. The first direction may in particular correspond to a transverse direction of the implant device perpendicular to a longitudinal direction along which the implant device may have a maximal length. Further, the first direction may correspond to a left-right (medial-lateral) direction of a patient receiving the implant device, in particular of a maxillary sinus of said patient, such that during implantation and/or once implanted, the first direction of the implant device may be aligned with said left-right direction.

In the folded state, the implant device has a greater curvature in the first direction than in the unfolded state. In other words, in the folded state the implant device may be curved in the first direction with a smaller radius of curvature than in the unfolded state. In another direction, in particular in a second direction, which may preferably be substantially perpendicular to the first direction, the implant device may be substantially flat irrespectively of whether the implant device is in the folded or in the unfolded state. "Curvature" may refer herein to curvature in the geometrical sense, i.e. to the amount by which a surface deviates from being a plane, as expressed for example in mathematical terms by a second derivative.

For example, an implant device according to the invention may be substantially planar or slightly curved/arched in the first direction in the unfolded state, and may be strongly curved, for instance rolled up in the first direction, i.e. wrapped around a direction perpendicular to the first direction, in the folded state.

The implant device of the invention is further plastically extendible in a second direction, which may in particular be different from the first direction. Preferably, the second direction may be substantially perpendicular to the first direction. For example, the first direction may correspond, as previously mentioned, to a transverse direction of the implant device and the second direction may correspond to a longitudinal direction perpendicular to the transverse direction, wherein the implant device may have a maximal length along the longitudinal direction. The second direction may correspond to an anterior-posterior (ventral-dorsal) direction of a patient to receive the implant device, in particular of a maxillary sinus of said patient, such that during implantation and/or once implanted, the second direction of the implant device may be aligned with said anterior-posterior direction.

The implant device of the invention is plastically extendible in the second direction from an unextended state to an extended state, in which the implant device has a longer extension in the second direction than in the unextended state. "Plastically" extendible may refer herein to the fact that, once extended to the extended state, in particular by moderate forces manually applicable by a human operator (e.g. a surgeon), be it with bare hands or with the aid of a proper medical tool, the implant device of the invention retains its new form and dimensions and it does not elastically compress back by itself to the unextended state, as would be the case of an elastic deformation. The implant device may be extendible by means of material stretching and/or material deformation.

According to the invention, the implant device is plastically extendible in the second direction to a length in the second direction equal to or greater than an aperture distance of a human maxillary sinus in an anterior-posterior direction, in particular of the maxillary sinus of a human adult, more specifically of the maxillary sinus of the recipient of the implant. Such distance in an anterior-posterior direction (also referred to in the medical literature as the "depth" or "length" of the maxillary sinus), naturally has a certain variability, for it may be different in different patients. For example, it is known to be larger for males than for females and it is also known to be different for different ethnic groups. Statistical deviations between left and right maxillary sinuses are also observed. However, the specific values vary within ranges that are known to surgeons and persons skilled in the art related to the field of the present invention, and that are sufficiently narrow and identifiable for the skilled person to implement the teachings of the present invention.

In particular, if an implant device is to be developed and/or manufactured for a specific population group of potential patients or for a specific individual patient, the implant device according to the present invention can be adapted accordingly to be extendible in the second direction to a length in the second direction equal to or greater than an aperture distance of the human maxillary sinus in an anterior-posterior direction for said population group (for example using a mean value or a minimal or maximal value) or for said individual patient.

As used herein the "aperture distance of a human maxillary sinus in an anterior-posterior direction" may in particular refer to an aperture distance of said human maxillary sinus in said anterior-posterior direction at a height within the maxillary sinus proximal to the roof of the maxillary sinus, i.e. proximal to the overlying orbital floor and to a cranial end of the maxillary sinus, which may correspond to a vertical (cranial-caudal) distance from the roof of the maxillary sinus of 10 mm or less, preferably of 5 mm or less.

In human adults, the aperture distance of the maxillary sinus in an anterior-posterior direction is typically in the range of 25 mm to 45 mm. Thus, the implant device of the invention may be extendible in the second direction to a length in the second direction of at least 25 mm, in particular to a length in the second direction of 25 mm to 55 mm, preferably of 28 mm to 48 mm. In other words, the implant device may have a maximal extension in the second direction in the aforesaid ranges, which may be adapted for a specific implant recipient or groups of target implant recipients.

Notably, the plastic extendibility of the implant device of the invention may cover any maximal length of the implant device in the second direction in the extended state suitable for reaching or exceeding said aperture distance of the maxillary sinus of a recipient of the implant in an anterior-posterior direction. The implant device of the invention, in particular the length thereof in the second direction, can be adapted to the individual anatomy of the recipient of the implant and thereby provide a personalised solution.

Thus, in the extended state, the length of the implant device in the second direction may be such that a first end of the implant device in the second direction may contact a posterior/anterior sidewall of the maxillary sinus of a human patient while a second (the other) end of the implant device in the second direction may contact an anterior/posterior sidewall of said maxillary sinus of said human patient. In other words, the dimension of the implant device in the second direction in the extended state is such that the implant device can entirely cover a separation distance between opposing sidewalls of the maxillary sinus of a human patient, formed by the respective anterior and posterior parts of the maxilla and/or the zygomatic bone, and can possibly exceed that distance.

Due to its being foldable in the first direction, the implant device of the invention can have a reduced spread in the first direction in the folded state. This allows inserting the implant device into an implantation site while the implant device is in the folded state, such that a reduced diameter or size of an insertion opening through which the implant device is to be inserted, for example an insertion opening provided by a surgeon in the lateral nasal wall of the patient receiving the implant, may be sufficient.

Further, due to its being plastically extendible up to an extension that is at least equal to a size of the maxillary sinus in the posterior/anterior direction, and to its ability to retain this extension, the implant device of the invention can be used for treating a damaged orbital floor, in particular an orbital blowout fracture, with reduced invasiveness. The length of the implant device in the second direction is such that the implant device can contact the bony sidewalls of the maxillary sinus at both ends of its extension in the second direction, which may in particular correspond to the anterior (ventral) and posterior (dorsal) sidewalls of the maxillary sinus, thereby fixating a position of the implant device by tension such that the implant device abuts against said sidewalls without the need of necessarily using more invasive fastening means such as screws or the like.

The implant device of the invention can hence be inserted into the maxillary sinus of a patient in the folded state (and in the unextended state) having reduced dimensions, for example through an insertion opening formed in the lateral nasal wall of the patient. Once it is received within the maxillary sinus, underneath the fractured orbital floor, the implant device can be arranged in the unfolded state and extended to the extended state such as to span a surface underneath the fractured orbital floor, in particular entirely covering a size of the maxillary sinus in the anterior-posterior direction (contrary for example to the implant plate known from the aforementioned document US 5,743,913, which is foreseen for covering only a partial portion of the orbital floor).

The implant device of the invention is specifically designed for this endonasal - in particular prelacrimal - approach for placing the implant device at the implantation site, which is less invasive than other known techniques based on accessing the orbital floor through the eye cavity (transconjunctival approach) or from the lower-lid region (subciliar approach, subtarsal approach, infraorbital approach), which typically require cranially displacing the content of the orbit including the eyeball and hence pose a risk to the integrity of the optic nerve.

Further, the prelacrimal approach for which the implant device of the invention is specifically designed can offer a surgeon better visibility of the implantation region by means of an optical monitoring instrument, such as a fiber-optic cannula or the like, which can be inserted into the implantation region using the same opening used for inserting the implant device and may offer very good visibility of the entire implantation region. In the aforementioned known implantation techniques, the visibility of the implantation region may depend on the position of the fracture in the orbital floor, such that a fracture located proximal to a posterior end of the maxillary sinus may be difficult to access visually and/or may require a considerable displacement of the content of the orbit.

As an additional advantage, the foldability in the first direction and the extendibility in the second direction allow the implant device of the invention to have reduced size and area prior to implantation and during implantation and to increase its size and area as appropriate once arranged within the maxillary sinus of the implant recipient close to the orbital floor fracture to be treated. This can allow, particularly in cases of orbital floor fractures of considerable extension, inserting into the region to be implanted an implant device sufficiently large for covering such large fractured zones of the orbital floor.

As to the foldability of the implant device in the first direction, it may be plastic in some embodiments and elastic in other embodiments.

If the implant device is plastically foldable in the first direction, the implant device may switch between the folded and the unfolded state and vice versa only when being correspondingly mechanically actuated upon, for example by an intervening surgeon with the aid of a surgery tool. Advantageously, this may allow manufacturing the implant device of one same material, since the same or similar plastic mechanical properties may be required by the material both in the first direction and in the second direction.

If the implant device is elastically foldable in the first direction, the implant device may switch from the folded state to the unfolded state by itself once mechanical forces (for example a wrapping force exerted by a surgeon, possibly using a corresponding tool, for inserting the implant device into the maxillary sinus of the patient) stop acting upon the implant device. The material properties of the implant device may be adapted such that the conversion from the folded state to the unfolded state may occur in an acceptable predefined time lapse, neither too slow for the implantation to be ineffective nor to quickly for the unfolding to be potentially dangerous. Advantageously, this may allow bringing the implant device into the maxillary sinus in the unfolded state and letting it unfold elastically without requiring any direct mechanical actuation upon the implant device for unfolding it, and hence possibly no use of a corresponding tool. Further, by unfolding elastically, the implant device of the invention may better adapt to the internal anatomy of the maxillary sinus of the patient. The elastic properties of the implant device may be implemented by directional material components, for example by elastically foldable mesh segments oriented in the first direction.

According to some embodiments, the implant device maybe extendible in the second direction to a length in the second direction 5% to 20% greater than an aperture distance of a human maxillary sinus, in particular of the patient receiving the implant or the targeted group of potential patients (e.g. a mean value), in an anterior-posterior direction, preferably 10% to 15% greater. This may allow the implant device to generate sufficient tension when abutting against the opposing posterior and anterior sidewalls of the maxillary sinus of the patient for providing a stable and durable implant capable of reliably supporting the orbital floor. When designing an implant device according to the invention for a specific individual patient or group of potential patients, known measurements of the corresponding aperture distances in anterior-posterior direction of the maxillary sinus to receive the implant can be relied upon for deciding on the extendibility of the implant device. For example, if an implant device is being manufactured for a patient presenting an aperture distance in anterior-posterior direction of the maxillary sinus of 36 mm, the implant device may be designed to be extendible in the second direction at least to a length of 43 mm.

Additionally or alternatively, the implant device may be bendable in the second direction, such that the implant device can simultaneously extend in the second direction to the extended state and bend/curve in the second direction. This may in particular imply that a curvature of the implant device in the second direction may be greater in the extended state than in the unextended state. This allows the implant device curving when extended within the maxillary sinus of the patient receiving the implant, in particular upwards (cranially) towards the orbital floor. As a consequence, a central part of the implant device may provide mechanical support to the fractured orbital floor while opposing ends of the implant device in the second direction provide mechanical fixation of the implant device to the bony sidewalls of the maxillary sinus. Advantageously, the possibility of reaching the orbital floor by bending/curving the implant devices eliminates the necessity of conforming the implant device to the orbital floor by applying direct pressure upon the fractured region of the orbital floor, thereby allowing a less-invasive fracture treatment.

Since the length of the implant device in the second direction may exceed the aperture distance of the maxillary sinus of the patient receiving the implant in an anterior-posterior direction and the implant device may be bendable in the second direction, the implant device may curve in the second direction when being extended to the extended state, in particular cranially. Once the ends of the implant device in the second direction reach the respective opposed sidewalls of the maxillary sinus, i.e. abut against the corresponding bones, the implant device may need to start curving as it extends further in order to adapt its shape with increasing length to the limited space available. Once the maximal extension of the implant device in the second direction is reached, the maximal curvature may also be reached and the implant device may rest in position providing stable and durable support to the orbital floor arranged over the maxillary sinus in which the implant device is received.

Thus, taking into account that the typical aperture distance of a human maxillary sinus in an anterior-posterior direction is in the range of 25 to 50 mm, in the extended state, the implant device may have a (curved) length in the second direction of 25 to 55 mm or more, for example of 32 mm, 38 mm, 43 mm or 58 mm. When curvedly arranged spanning the maxillary sinus of the patient underneath the fractured orbital floor, a vertical distance in the cranial-caudal direction covered by the implant device, i.e. a vertical distance between an uppermost point of the implant device, which may correspond to a portion of the implant device contacting the orbital floor, and a lowermost point of the implant device, which may correspond to a portion of the implant device contacting the maxilla, maybe of 1 mm to 8 mm.

In some embodiments, in the unextended state the implant device may have a length in the second direction 10% to 50% smaller than in the extended state, preferably 20% to 30% smaller. The relative reduction of length in the second direction of the implant device in the unextended state, which the implant device generally has prior and during the implantation process, with respect to the extended state, which the implant device generally has once implanted, reduces the handling and implantation complexity of the implant device and further reduces invasiveness.

Preferably, the implant device may have, in the unextended state, a length in the second direction smaller than an aperture distance of a human maxillary sinus in an anterior-posterior direction, in particular of the maxillary sinus of a human adult, more specifically of the maxillary sinus of the recipient of the implant. Thus, when the implant device is inserted into the implantation site, its length in the second direction may be such that the implant device does not contact any of the sidewalls of the maxillary sinus before being extended to the extended state. This can allow a less invasive implantation. For example, in the unextended state, the implant device may have a length in the second direction of 15 mm to 45 mm, preferably of 20 mm to 40 mm.

In preferred embodiments, the implant device may comprise at least one extensible portion and at least one stabilisation portion. The at least one extensible portion may be plastically extendible in the second direction to said length in the second direction equal to or greater than an aperture distance of a human maxillary sinus in an anterior-posterior direction. Thus, the implant device may be extensible in the second direction by the extensible portion. The at least one stabilisation portion may have a reduced extensibility in the second direction compared to the at least one extensible portion. For example, an extensibility of the at least one stabilisation portion in the second direction may be 50% smaller, 75% smaller, 100% smaller or 200% smaller than an extensibility of the at least one extensible portion in the second direction. The at least one stabilisation portion may possibly be effectively rigid in the second direction, meaning that the extensibility thereof in the second direction may be negligible compared to an extensibility of the at least one extensible portion.

Thus, when the implant device is extended from the unextended state to the extended state, the extension in the second direction may be mostly or completely implemented by a corresponding extension of the at least one extensible portion, whereas the at least one stabilisation portion may extend only slightly or even not extend at all. For instance, the implant device may be divided along the second direction in a central extensible portion arranged between two stabilisation portions. The at least one extensible portion may provide the required plastic extensibility in the second direction for the implant device to adapt to the length of the corresponding maxillary sinus of the patient receiving the implant in an anterior-posterior direction, whereas the at least one stabilisation portion may provide the required stability for the implant device to stay in place once implanted and to support the fractured orbital floor.

According to preferred embodiments, the implant device may comprise a first support edge and a second support edge arranged at opposed ends of the implant device in the second direction. The first and second support edges are configured for abutting against opposing sidewalls of a cavity, in particular of a human maxillary sinus, more particularly of the maxillary sinus of a recipient of the implant, such that the implant device is held by tension against said sidewalls. The first and second support edges are hence configured for acting as mechanical supporting structures of the implant device of the invention when placed within the maxillary sinus. Instead of requiring specific fastening means insertable into surrounding bone tissue such as screws or the like, the implant device of the invention can be fastened to the surrounding bony sidewalls of the maxillary sinus by tension by means of the dedicated first and second support edges. The first and second support edges maybe blunt, for example rounded. By being blunt, the first and second support edges may reduce the risk of accidental damage to surrounding tissue during implantation. However, the first and second support edges need not be blunt and may comprise non-blunt anchoring features such as spikes, serrations, hooks or the like, for improving a fixation of the first and second support edges to the sidewalls of the maxillary sinus of the recipient of the implant. Notably, eventual small tissue injuries caused by such anchoring features in the sidewalls of the maxillary sinus of the recipient of the implant typically heal in an acceptable time.

With reference to the aforementioned embodiments in which the implant device comprises at least one extensible portion and at least one stabilisation portion, the first and second support edges may be arranged in a first stabilisation portion and in a second stabilisation portion of the at least one stabilisation portion, respectively. For example, the implant device may be divided along the second direction in a central extensible portion arranged between two stabilisation portions, wherein the first support edge may be arranged in one of the two stabilisation portions and the second support edge may be arranged in the other one of the two stabilisation portions.

In preferred embodiments of the invention, the implant device may be a mesh-like device comprising at least one first mesh segment and at least one second mesh segment. The first and second mesh segments may form a lattice or matrix structure. The at least one first mesh segment may be foldable in the first direction and the at least one second mesh segment may be plastically extendible in the second direction. Thus, the at least one first mesh segment may be configured for implementing the foldability of the implant device in the first direction and the at least one second mesh segment may be configured for implementing the plastic extendibility of the implant device in the second direction. The implant device of the invention may hence be folded and unfolded in the first direction between the folded state and the unfolded state by correspondingly deforming, bending and/or stretching/compressing the at least one first mesh segment and may be extended from the unextended state to the extended state by correspondingly deforming, bending and/or stretching the at least one second mesh segment.

The at least one first mesh segment may comprise a plurality of first mesh segments having different forms, structures, material compositions and/or orientations in order to allow for different ways of folding and unfolding the implant device in the first direction. The at least one second mesh segment may comprise a plurality of second mesh segments having different forms, structures, material compositions and/or orientations in order to allow for different ways of extending the implant device in the second direction. This may allow better adapting the implant device of the invention to the specific anatomy of the maxillary sinus of an individual patient, accounting for the regular geometry of the maxillary sinus and/or of the orbital floor. For example, when unfolding the implant device within the maxillary sinus, it may be necessary to provide a greater curvature at one part of the implant device than at another part of the implant device and/or, when extending the implant device into the unextended state, it may be necessary to provide a greater length or curvature at one part of the implant device than at another part of the implant device.

As to the dimensions of the implant device in the first direction, according to some embodiments, the implant device may have, in the unfolded state, a width in the first direction equal to or smaller than an aperture distance of a human maxillary sinus in a left-right direction, in particular of the maxillary sinus of a human adult, more specifically of the maxillary sinus of the recipient of the implant. In human adults, the aperture distance of the maxillary sinus in a left-right direction is typically in the range of 20 mm to 35 mm. Thus, the implant device of the invention may have a width in the first direction of less than 35 mm, preferably in the range of 10 mm to 30 mm. Notably, the width of the implant device of the invention can be adapted to the individual anatomy of the recipient of the implant and thereby provide a personalised solution. The width of the implant device, i.e. the dimension of the implant device in the first direction, may hence be such that, once implanted in position, left and right edges of the implant device arranged at opposed ends of the implant device in the first direction do not contact any sidewalls of the maxillary sinus of the implant recipient, i.e. are floating.

In preferred embodiments of the invention, in the folded state, the implant device is wrapped around the second direction with a wrapping radius of 10 mm or less, preferably of 8 mm or less, more preferably of 5 mm or less. The wrapping radius may for example be in the range of 5 mm to 8 mm. In other words, in the folded state, the implant device may approximately resemble a cylinder with a central axis extending in the second direction, being curved in the first direction with the aforesaid wrapping radius, and having a diameter of 20 mm or less, preferably of 16 mm or less, more preferably of 10 mm or less. This can allow the implant device of the invention to be insertable into the maxillary sinus of a patient through an insertion opening, in particular an insertion opening formed in the lateral nasal wall of the patient, which opening can have a diameter of 20 mm or less, preferably of 16 mm or less, more preferably of 10 mm or less. Preferably, the diameter of the implant device in the folded state is smaller than the diameter of the insertion opening through which the implant device is to be inserted, in particular at least 10% to 40% smaller. The possibility of folding the implant device to with such a reduced wrapping radius may leave enough space in the insertion opening for a further tool to be simultaneously inserted therethrough, in particular an exploration tool, for example allowing an intervening surgeon to further use the same insertion opening for passing an endoscope therethrough in order to monitor the implantation site during implantation.

In some preferred embodiments, the implant device of the invention may comprise a first manipulation eyelet and a second manipulation eyelet mutually separated in the second direction. The implant device can be plastically extended in the second direction by mutually separating the first and second manipulation eyelets. The manipulation eyelets can hence serve as provisional fixation or grip points at which a surgical tool can exert a force upon the implant device for extending the implant device from the unextended state to the extended state. The first and second manipulation eyelets may be aligned with each other along the second direction and maybe mutually separated in the second direction by a separation distance of 10 mm to 20 mm. Each of the first and second manipulation eyelets may have an aperture - for example a diameter in the case of circular manipulation eyelets - of 1 mm to 2 mm, although the first and second manipulation eyelets need not have circular shape. Preferably, the first and second manipulation eyelets may be coplanar with the rest of the implant device, such that they do not protrude from the plane of the implant device.

With reference to the aforementioned embodiments in which the implant device comprises at least one extensible portion and at least one stabilisation portion, the first and second manipulation eyelets may be arranged in a first stabilisation portion and in a second stabilisation portion of the at least one stabilisation portion, respectively. For example, the implant device may be divided along the second direction in a central extensible portion arranged between two stabilisation portions, wherein the first manipulation eyelet may be arranged in one of the two stabilisation portions and the second manipulation eyelet may be arranged in the other one of the two stabilisation portions.

According to some embodiments, in the unfolded state, the implant device of the invention may approximately have the shape of a planar parallelogram, for example a rectangle or a square, preferably with rounded edges and/or rounded corners, wherein the first and second directions may respectively correspond to the width and the length of the parallelogram. Such regular configuration of the implant device of the invention may simplify the manufacturing and handling thereof. "Planar" may in particular refer herein to a sheet-like configuration of the implant device, wherein a thickness of the implant device may be considerably smaller than a length and/or a width of the implant device. For example, the implant device may have a thickness in a third direction perpendicular to both the first direction and a second direction of 0.1 mm to 0.5 mm.

In preferred embodiments of the invention, the implant device may comprise or be made of a biocompatible material, preferably a metallic material. The implant device of the invention may for example be made of or comprise titanium or an alloy thereof (e.g. Ti6Al4V), surgical steel, a cobalt-chromium alloy, a nickel-titanium alloy (e.g. Nitinol) or any combination thereof. Such material composition prevents undesired secondary effects related to implant incompatibility and/or rejection and allows the implant device of the invention to have sufficient rigidity and mechanical stability for effectively supporting the fractured orbital floor from below and for retaining its final shape once the implantation process is finalised.

A further aspect of the invention relates to an implantation tool for implanting an implant device according to the invention, in particular according to any of the previously described embodiments, at an orbital floor fracture/within the maxillary sinus of a patient. The implantation tool is a medical/surgical tool specifically adapted for the implantation of the implant device of the invention at an orbital floor of a subject (particularly on a roof of the maxillary sinus) for treating an orbital floor fracture. The implantation tool may in particular be adapted for implanting an implant device according to the invention via an insertion opening in the lateral nasal wall of the subject, i.e. through the previously described prelacrimal approach. The subject refers herein to the implant recipient.

The implantation tool comprises a holding part and an insertion part. The holding part is configured for being held an operated by a user, for example by a surgeon or by a surgery robot. The holding part may in particular be configured for being hand-held, possibly having a corresponding ergonomic design, for example having a grip-like and/or pistol-like configuration. The insertion part, which may be connected or connectable to the holding part, for example partly receivable into the holding part, is configured for being inserted into the body of the subject. Thus, the holding part is the part of the implantation tool allowing a human or robot operator of the implantation tool to hold and operate the device, in particular from the exterior of the body of the subject, while the insertion part is the part of the implantation tool designed for being inserted into the body of the subject during implantation.

The insertion part comprises an insertion tip. The insertion tip is configured for holding the implant device in the folded state and in the unextended state. Thus, in an initial state prior to implantation, the implant device can be received on the insertion tip in the folded and unextended states, such that the implant device can be inserted into the body of the subject having a reduced size and volume. The insertion tip further comprises an unfolding mechanism and an extending mechanism. The unfolding mechanism is configured for unfolding the implant device from the folded state to the unfolded state. The extending mechanism is configured for extending the implant device from the unextended state to the extended state. Thus, after being received on the insertion tip in the folded and unextended states, the implant device of the invention can be inserted into the implantation site, i.e. into the proximity of the roof of the maxillary sinus of the subject. Once there, the unfolding mechanism of the implantation tool can be used for setting/converting the implant device into the unfolded state and the extending mechanism can be used for setting/converting the implant device into the extended state.

The holding part of the implantation tool further comprises a control mechanism configured for controlling the unfolding mechanism and the extending mechanism. The control mechanism hence allows the operator of the implantation tool of the invention controlling the operation of the unfolding mechanism and of the extending mechanism from the outside of the body of the subject receiving the implant, thereby governing, for example, the position of the implant device within the maxillary sinus of the subject when the implant device is unfolded to the unfolded state and/or extended to the extended state.

The implantation tool of the invention is hence specifically designed for the implantation of an implant device according to the first aspect of the invention, in particular for inserting the implant device into the maxillary sinus of a patient in the folded state and in the unextended state having reduced dimensions, in particular through an endonasal approach, preferably a prelacrimal approach, for example via an insertion opening formed in the lateral nasal wall of the subject. The implantation tool is configured for introducing the implant device into the maxillary sinus, underneath the damaged orbital floor, and for then setting the implant device in the unfolded state and in the extended state such as to span a surface underneath the damaged orbital floor and hold the content of the orbit, in particular entirely covering a size of the maxillary sinus in the anterior-posterior direction. As previously mentioned, the endonasal approach proves to be less invasive than other known techniques based on accessing the orbital floor through the eye cavity (transconjunctival approach) or from the lower-lid region (subciliar approach, subtarsal approach, infraorbital approach), which typically require cranially displacing the content of the orbit including the eyeball and hence pose a risk to the integrity of the optic nerve.

In preferred embodiments, the insertion part maybe detachably connectable with the holding part. The insertion part may hence be detached from the holding part, for example for being replaced by another appropriate insertion part, such that the holding part may be repeatedly re-used for different implantation operations. The insertion part may for example be detachably connectable with the holding part via a bayonet mechanism or the like. Additionally or alternatively, the insertion tip may be detachably connectable with the insertion part, for example for being replaced by another appropriate insertion part, such that the holding part may be repeatedly re-used for different implantation operations.

According to preferred embodiments, the insertion part may comprise an elongated shaft, which may preferably be rigid. The shaft may extend from one longitudinal end of the shaft proximal to the holding part to another longitudinal end of the shaft comprising the insertion tip or being connected thereto. The shaft may be specifically designed for being inserted through an opening formed in the body of the subject, in particular in a lateral nasal wall of the subject, for inserting therethrough the implant device received on the insertion tip. Preferably, the shaft may have a length of 3 cm to 12 cm, more preferably of 4 cm to 8 cm. This can be an optimal length range of the shaft for comfortably operating the implantation tool from the exterior of the body of the patient while having sufficiently control and handling possibilities. Additionally or alternatively, the shaft may have a diameter of2 mm to 10 mm, preferably of 3 mm to 6 mm, which can allow inserting the insertion tip holding the implant device through an insertion opening, in particular an insertion opening formed in the lateral nasal wall of the patient, having a diameter of 20 mm or less, preferably of 16 mm or less, more preferably of 10 mm or less. Advantageously, the diameter of the shaft allows simultaneously passing the shaft and a surgical monitoring tool such as an endoscope, a fiber-optic cannula or the like through such an insertion opening.

Optionally, the insertion tip may be configured for holding the implant device angled with respect to the longitudinal direction of the shaft, preferably at an angle of 15° to 60°, more preferably of 20° to 45°. This may allow easily positioning the implant device throughout the implantation process according to the anatomy of the subject.

In some embodiments, the insertion tip may be dimensioned such that the insertion tip does not contact any sidewalls of a maxillary sinus of the implant recipient during the implantation process.

In preferred embodiments, the unfolding mechanism may comprise one or more flaps movable in a direction corresponding to the first direction of the implant device (received on the insertion tip). The one or more flaps may in particular comprise at least two flaps arrangeable substantially coplanar for unfolding the implant device. The one or more flaps may in particular be swivable. For example, the implantation tip may comprise two lateral swivable flaps configured for receiving the implant device in the folded state thereon when the flaps are closed, i.e. aligned with a surface of the shaft without substantially protruding from it. When the insertion tip reaches the implantation side, the control mechanism can be operated to open the flaps, such that the flaps swivel horizontally opening up separating from the shaft, thereby unfolding the implant device arranged thereon. In the completely open position of the flaps, the two flaps may be substantially coplanar with each other, such that the implant device is substantially planar.

According to preferred embodiments, the extending mechanism may comprise at least one first fastener and at least one second fastener for fastening and extending the implant device. The at least one first fastener and the at least one second fastener may be movable in a direction corresponding to the second direction of the implant device. Thus, by holding the implant device at at least two different positions and by being movable in a direction corresponding to the second direction of the implant device, the extending mechanism can allow controllably extending the implant device to the extended state. The at least one first fastener and the at least one second fastener may be configured for fastening the implant device at two respective different positions along the second direction of the implant device, in particular within an extensible portion thereof. In embodiments in which the implantation device comprises first and second manipulation eyelets, the at least one first fastener and the at least one second fastener may be configured for fastening the implant device by the first and second manipulation eyelets, respectively. The at least one first fastener and the at least one second fastener may for example be configured as fastening hooks or as fastening protrusions. The at least one first fastener and the at least one second fastener may be retractable such that they can release the implant device and be moved back to a retracted position, preferably such that they are aligned with an exterior surface of the shaft, such that the implantation tool can be easily and safely extracted from the body of the subject after placing the implant device in the final implantation position.

Preferably, the at least one first fastener and the at least one second fastener may be movable in said direction corresponding to the second direction of the implant device for an extension distance corresponding to a length difference of the implant device in the extended state with respect to the unextended state. For example, if the implant device is configured for having a length in the unextended state of 25 mm and a length in the extended state of 38 mm, a first fastener may be movable in said direction corresponding to the second direction of the implant device with respect to a second fastener by 13 mm.

A further aspect of the invention relates to an implantation system for treating an orbital floor fracture of a subject. The implantation system comprises an implant device according to the invention, in particular according to any of the previously described embodiments, and further comprises an implantation tool according to the secondly mentioned aspect of the present invention, in particular according to any of the previously described embodiments thereof, for implanting the implant device at the orbital floor fracture.

A related method of providing an implant, in particular an implant device according to the first aspect of the invention, at a site of an orbital floor fracture of a subject will be described now. The method comprises providing an implant according to any the previously described embodiments of the invention and inserting the implant device into the body of the subject with the implant device arranged in the unextended state and in the folded state to arrange the implant device at the site of the orbital floor fracture. The method further comprises unfolding the implant device to the unfolded state and extending the implant device to the extended state.

The implant device may be inserted into a maxillary sinus of the subject through an insertion opening formed in the lateral nasal wall of the subject.

Extending the implant device to the extended state may comprise extending the implant device such that the implant device extends throughout the maxillary sinus of the subject in an anterior-posterior direction with the implant device resting on opposed sidewalls of the maxillary sinus of the subject and contacting the orbital floor from below.

Extending the implant device to the extended state may comprise curving the implant device in the second direction towards the overlying orbital floor.

Preferably, the implant device may be extended to the extended state such that a length of the implant device in the second direction exceeds an aperture distance of the maxillary sinus of the subject in an anterior-posterior direction by 5% to 20%, more preferably by 10% to 15%, in particular with first and second support edges of the implant device arranged at respective ends of the implant device along the second direction thereof resting on respective opposed sidewalls of the maxillary sinus and with the implant device being convexly curved in the second direction towards the overlying roof of the maxillary sinus and with a maximally curved portion of the curved implant device (i.e. with a vertex of the curved implant device) supporting the overlying orbital floor (the overlying roof of the maxillary sinus), such that the implant device is in a bridge-like configuration providing reliable and durable support to the fractured orbital floor of the subject.

The method may further comprise selectively extending, bending and/or deforming portions of the implant device in order to adapt the implant device to the anatomy of the orbital floor of the subject.

The method may be performed using an implantation system according to the second aspect of the invention, i.e. using the previously described implantation tool for implanting the implanting device.

### BRIEF DESCRIPTION OF THE FIGURES

- Fig. 1: shows a schematic illustration of a top view of an implant device according to the invention in the unfolded state and in the unextended state.
- Fig. 2: shows a schematic illustration of a top view of the implant device of Fig. 1 in the unfolded state and in the extended state.
- Fig. 3: shows a schematic illustration of a side view of the implant device of Figs. 1 and 2 in the folded state.
- Fig. 4: shows a schematic illustration of a side view of the implant device of Figs. 1 and 2 in the unfolded state.
- Fig. 5: shows a schematic illustration of a side view of an implantation tool according to the invention.
- Fig. 6: shows a schematic illustration of a top view of the implantation tool of Fig. 5.
- Fig. 7: shows a schematic illustration of a perspective view of the implantation tip of an implantation tool according to the invention, wherein an implant device is received on the implantation tip in the folded state and in the unextended state.
- Fig. 8: shows a schematic illustration of a perspective view of the implantation tip of Fig. 7, wherein the implant device is received on the implantation tip in the unfolded state and in the unextended state.
- Fig. 9: shows a schematic illustration of a top view of the implantation tip of Figs. 7 and 8, wherein the implant device is received on the implantation tip in the unfolded state and in the extended state.
- Fig. 10: is a schematic illustration of a method of providing an implant device according to the invention on an orbital floor fracture of a subject.
- Fig. 11: is a schematic illustration of the position of the implant device of Fig. 10 during and after implantation within the maxillary sinus of the subject in an anterior-posterior direction.

### DETAILED DESCRIPTION OF PREFERRED EMBODIMENTS OF THE INVENTION

For the purposes of promoting an understanding of the principles of the invention, reference will now be made to specific preferred embodiments illustrated in the drawings, and specific language will be used to describe the same. It will nevertheless be understood that no limitation of the scope of the invention is thereby intended, such alterations and further modifications in the illustrated apparatus and such further applications of the principles of the invention as illustrated therein being contemplated as would normally occur now or in the future to someone skilled in the art to which the invention relates within the scope defined by the claims.

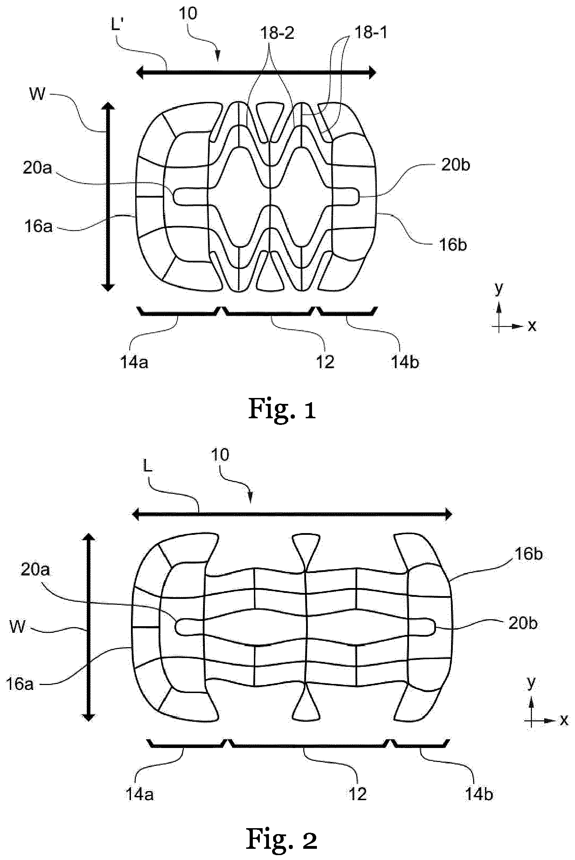

Figs. 1 and 2 show schematic top views of an implant device 10 according to an embodiment of the invention. The implant device 10 is a mesh-like laminar object approximately having the shape of a rectangle with rounded corners, which in the unfolded state is, as shown in Fig. 1 and 2, substantially planar. In Fig. 1, the implant device 10 is shown in the unfolded state and in the unextended state. In this state, the implant device 10 has a width W in a first direction y and a length L' in a second direction x perpendicular to the first direction y.

In the embodiment shown, the implant device 10 is divided along the second direction x in a central extensible portion 12 and two lateral stabilisation portions 14a, 14b arranged to the left and to the right of the central extensible portion 12, respectively. In the second direction x, the implant device 10 extends from a first support edge 16a, arranged at one longitudinal end of the implant device 10 in the stabilisation portion 14a, and a second support edge 16b, which is arranged at the other longitudinal end of the implant device 10 in the second direction x, in the other stabilisation portion 14b.

In each of the stabilisation portions 14a, 14b, the implant device 10 comprises a respective manipulation eyelid 20a, 20b, which are each arranged at different positions of the implant device 10 in the second direction x. In the embodiment shown, the manipulation eyelets 20a, 20b are aligned with each other with respect to the first direction y on a central axis of symmetry of the implant device 10.

The implant device 10 of the embodiment shown is made of titanium. Thanks to this material composition and to the mesh-like configuration, the implant device 10 is plastically extendible in the second direction x from the unextended state shown in Fig. 1, in which the implant device has a length L' in the second direction x, to an extended state, which is shown in Fig. 2, in which the length of the implant device in the second direction x is extended to a longer length L, i.e. L> L'.

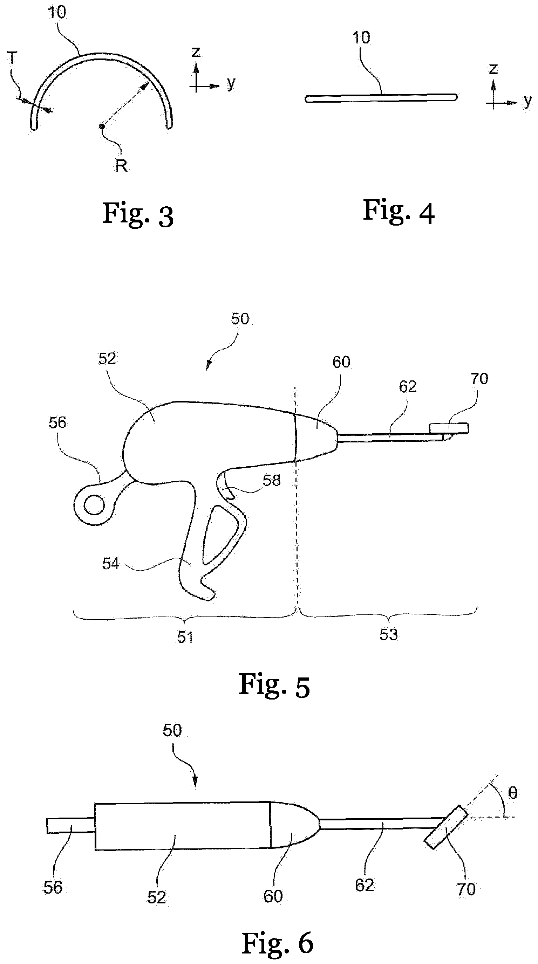

Further, the implant device 10 is foldable in the first direction y, in particular due to first mesh segments 18- which are foldable in the first direction y. In Fig. 1, only first mesh segments 18-1 in the extensible portion 12 are exemplarily indicated. However, the implant device 10 can comprise flexible first mesh segments also in the stabilisation portions 14a and 14b. Therefore, the implant device 10 can be folded and unfolded between the unfolded state that is represented in Figs. 1 and 2 in the xy-plane and also in Fig. 4 in the zy-plane, and a folded state that is represented in Fig. 3 in the zy-plane. As can be seen by comparing Figs. 3 and 4, in the folded state (see Fig. 3), the implant device 10 has a greater curvature in the first direction y than in the unfolded state (see Fig. 4). In the unfolded state represented in Fig. 4, the implant device 10 has a substantially planar or substantially zero-curvature configuration, whereas in the folded state shown in Fig. 3, the implant device has a substantially circular curvature characterised by a radius of curvature R, which can for example be 5 mm, for a width W of the implant device 10 of 18 mm. Also indicated in Fig. 3 is the thickness T of the implant device 10, which can be 0.1 mm to 0.4 mm in some embodiments.

As seen by comparing Figs. 1 and 2, the second mesh segments 18-2 arranged in the extensible portion 12 of the implant device 10 can deform and/or stretch in the second direction x in order to lengthen the implant device 10 in the second direction x, while other mesh segments arranged in the stabilisation portions 14a, 14b remain without any substantial change as to their extension in the second direction x.

Figs. 5 and 6 illustrate schematic views of an implantation tool 50 according to embodiments of the invention. An implantation system according to the invention can include the implantation tool 50 and an implant device like the implant device 10 shown in Figs. 1 to 4.

Fig. 5 shows a side view of the implantation tool 50, while Fig. 6 shows a top view. The implantation tool 50 is a surgical tool specifically configured for implanting the implant device 10 at the site of an orbital floor fracture. The implantation tool 50 comprises a holding part 51 and an insertion part 53. The holding part 51 has a pistol-like ergonomic form and is configured for being hand-held by a human operator, in particular by a surgeon. The holding part 51 comprises a main body 52, a grip portion 54 that can be comfortably hand-held by the human operator, a control mechanism 56 configured as a lever mechanism and a safety trigger 58. When the implantation tool 50 is hand-held by the operating user, the hand of the user can use the grip portion 54, such that the dumb of said hand can be used for operating the control mechanism 56 and the index finger can be used for operating the safety trigger 58, which unblocks the control mechanism 56 when being pulled.

The insertion part 53 is the part of the implantation tool 50 configured for being partly introduced into the body of a subject that is to receive the implant device. The insertion part 53 is detachably connected with the holding part 51 by means of a bayonet mechanism (not shown) that is implemented in the connection part 60 that connects the insertion part 53 to the holding part 51.

The insertion part 53 further comprises at a rigid elongated shaft 62, which extends from a proximal longitudinal end of the shaft 62 that is proximal to the holding part 51 and is connected to the connection part 60, to a distal longitudinal end of the shaft 62 that is connected to the insertion tip 70. The shaft 62 extends from its proximal longitudinal end to its distal longitudinal end having a length of 5 cm and a diameter of 4 mm.

As seen in Fig. 6, the insertion tip 70 is arranged angled with respect to the shaft 62 forming a relative angle θ, which can for example be in the range of 15° to 45°.

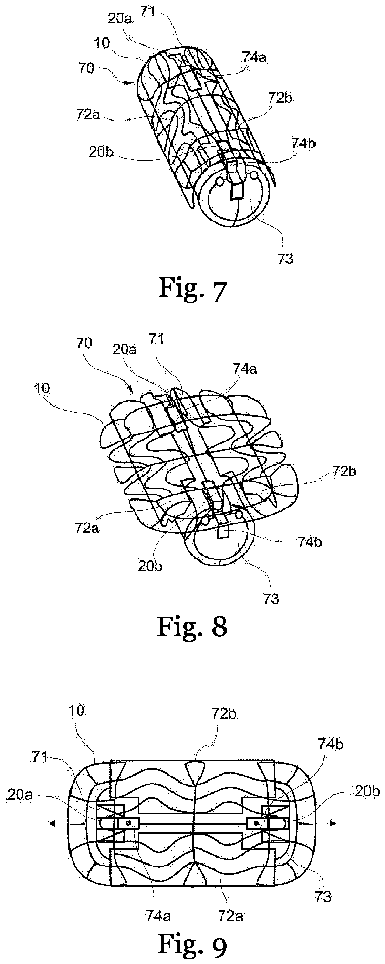

Figs. 7 to 9 show detailed views of the insertion tip 70 and illustrate the operation thereof. The insertion tip 70 is the part of the implantation tool 50 configured for receiving the implant device 10 thereon. The insertion tip 70 extends longitudinally from a first end 71 proximal to the holding part 51 (in particular when the angle θ shown in Fig. 6 is 0°) and a second end 73 distal with respect to the holding part 51. The implant device 50 comprises an unfolding mechanism, which in the exemplary embodiment shown is implemented by a pair of flaps 72a, 72b that are movable in the radial direction of the insertion tip 70, which corresponds to the first direction of the implant device 10 (the first direction y shown in Figs. 1 and 2). The flaps 72a, 72b are longitudinally (axially) arranged on opposite sides of the insertion tip 70.

Fig. 7 shows a situation in which the flaps 72a, 72b of the unfolding mechanism are closed, allowing the implant device 10 to be received on the insertion tip 70 in the folded state represented in Fig. 3. Fig. 8 represents a situation in which the flaps 72a, 72b of the unfolding mechanism open up, i.e. are swivelled open by correspondingly operating the control mechanism 56, thereby unfolding the implant device 10 to the unfolded state, such that the implant device 10 takes up the substantially planar configuration illustrated in Fig. 4.

In Figs. 7 and 8 the implant device 10 is in the unextended state represented in Fig. 1. The insertion tip 70 further comprises an extension mechanism implemented as a pair of hooks 74a, 74b that are arranged at different longitudinal (axial) positions of the insertion tip 70 and configured for engaging in one of the manipulation eyelets 20a, 20b of the implant device 10, respectively, thereby fixating the implant device 10 onto the insertion tip 70 of the implantation tool 50. As shown in Fig. 9, when the hooks 74a, 74b are moved apart in the longitudinal direction of the insertion tip 70, i.e. in a direction corresponding to the second direction of the implant device 10 (cf. second direction x illustrated in Figs. 1 and 2) by correspondingly operating the control mechanism 56, the implant device 10 is extended from the unextended state to the extended state.

The length in the second direction to which the implant device 10 is extended can be controlled by adjusting the distance by which the hooks 74a, 74b are movable or move apart from each other and/by adjusting an operation of the control mechanism 56. The hooks 74a, 74b can be configured as retractable elements that can be inwardly retracted into the surface of the insertion tip 70 in order not to protrude from the surface of the insertion tip 70 so as to unleash the implant device 10, in particular once it has been arranged in the unfolded state and in the extended state.

The operation of the flaps 72a, 72b and of the hooks 74a, 74b can be controlled by different settings of the control mechanism 56. In the embodiment of the implantation tool 50 illustrated in Figs. 5 and 6, the control mechanism 56 for controlling both the unfolding mechanism and the extension mechanism of the insertion tip 70 is configured as a single lever mechanism 56. However, in other embodiments, the unfolding mechanism and the extension mechanism may be controlled using respective mechanisms comprised in the control mechanism 56 of the implanting tool 50.

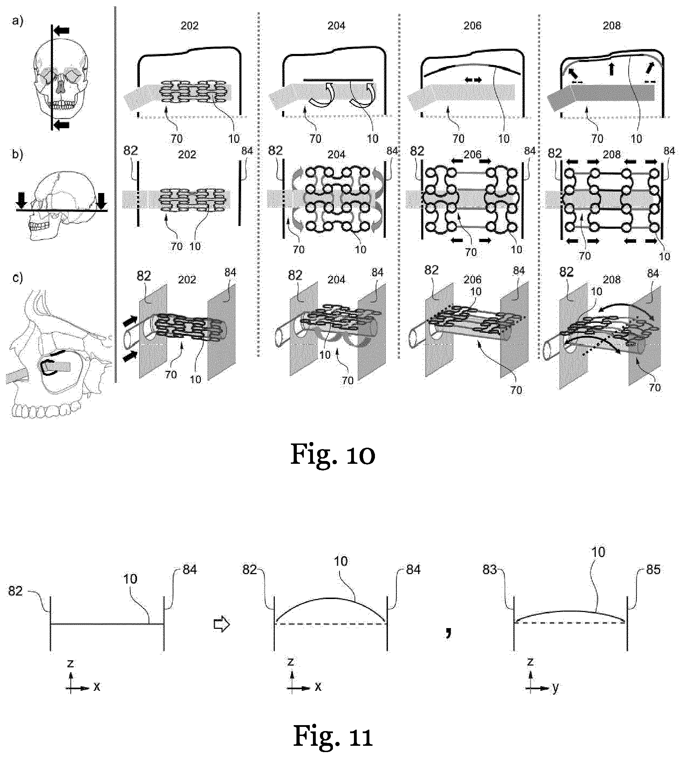

Fig. 10 schematically illustrates a method of providing an implant at a site of an orbital floor fracture of a subject using an implantation system according to the invention including an implantation tool 50 for arranging the implant device 10 at the site of the orbital floor fracture. Fig. 10a schematically illustrates different steps 202 to 208 of the method as seen from a lateral perspective, i.e. in a left-right direction as indicated in the leftmost drawing of Fig. 10a. Fig. 10b shows the same sequence of steps 202 to 208 from a cranial perspective as indicated in the leftmost drawing of Fig. 10b and Fig. 10c shows a perspective view of the same sequence of steps 202 to 208 as indicated in the leftmost drawing of Fig. 10c.

In a step 202, the implant device 10 received on the insertion tip 70 of the implantation tool 50 in the unextended state and in the folded state is inserted into the maxillary sinus of the subject through an insertion opening formed in the lateral nasal wall of the subject, i.e. according to the so-called prelacrimal approach. Thereby, the implant device reaches the interior of the maxillary sinus and approaches the fractured orbital floor from below.

In step 204, when the implant device 10 is received within the maxillary sinus of the subject, the control mechanism 56 can be used for operating the unfolding mechanism, for example for opening up the flaps 72a, 72b shown in the embodiment illustrated in Figs. 7 to 9, in order to unfold the implant device 10 to the unfolded state. Thereupon, the control mechanism 56 can be used for operating the extension mechanism, for example for moving the hooks 74a, 74b of the embodiment illustrated in Figs. 7 to 9 apart from each other, in order to extend the implant device 10 to the extended state in step 206.

The dimensions of the implant device 10, in particular the length thereof along the second direction x (cf. Figs. 1 and 2), can be adapted to the anatomy of the maxillary sinus of the subject receiving the implant device 10. For example, if a distance between opposite side walls 82, 84 of the maxillary sinus of the subject in an anterior-posterior direction is of 37 mm, the implant device 10 may be designed to have a length in the second direction x in the unextended state (cf. length L' in Fig. 1) of 32 mm, such that in step 204 and before step 206, the implant device 10 does not contact any sidewalls of the maxillary sinus of the subject. Further, the implant device 10 may be designed to be extendible by 10 mm to a length in the second direction x in the extended state (cf. length L in Fig. 2) of 42 mm, such that after carrying out step 206, the first support end 16a of the implant device 10 contacts the anterior sidewall 82 of the maxillary sinus while the second support edge 16b contacts the posterior sidewall 84 of the maxillary sinus. The implant device 10 is thereby held in position against the sidewalls 82, 84 of the maxillary sinus by the pressure exerted by the first and second support ends 16a, 16b.

In each of the stabilisation portions 14a, 14b, the mesh segments are configured and arranged in such a manner that the distribution of forces within the implant device 10 flows straight through the implant device 10, thereby providing rigidity and durability to a support structure formed by the implant device 10, in particular to the orbital floor, when the orbital floor is supported by the implant device 10.

When the implant device 10 is extended to the extended state, the mesh segments in the extensible portion 12 reposition extending their length in the second direction, thereby increasing their rigidity and closing the gaps formed in the mesh structure of the implant device 10, thereby providing a more homogeneous support structure for the orbital floor (see Figs. 1 and 2).

The implant device 10 is further bendable in the second direction x. Since the implant device 10 is extendible to a maximum length of 42 mm, which is longer than the distance of 37 mm between the side walls 82, 84 of the maxillary sinus of the subject, in the course of reaching its maximum length in the second direction, the implant device 10 curves upwards towards the fractured orbital floor that overlies the maxillary sinus. Notably, the implant device 10 cannot curve downwards instead - away from the fractured orbital floor overlying the maxillary sinus - due to the presence of the implantation tip 70 on which the implant device 10 is arranged. This curving of the implant device 10 causes a central portion of the implant device 10 to reach the fractured orbital floor, thereby providing support to the orbital floor, while the first and second support edges 16a, 16b abut against respective side walls 82, 84 of the maxillary sinus and hold the implant device 10 in position. Thus, the implant device 10 takes out a bridge-like configuration within the maxillary sinus of the subject providing durable and reliable support to the orbital floor of the subject. This is illustrated in detail in Fig. 11.

As seen in Fig. 11, once extended to the extended state, the implant device 10 extends in a bridge-like manner between the opposing sidewalls 82 and 84 of the maxillary sinus. The curvature in the x-direction can be additional to a remaining curvature in the y-direction if the implant device 10 does not flatten up completely when converting from the bent state to the unbent state, such that the implant device 10 can take up the form of a circular paraboloid exhibiting a convexity (with respect to the fractured orbital floor) both in the first direction corresponding to the anterior-posterior direction and in the second direction corresponding to the left-right direction.

As a consequence of step 206, the implant device 10 is correctly positioned for providing structural support to the fractured orbital floor without having to apply any direct force against the fractured orbital floor in order to adapt the implant device 10 to the shape thereof. However, in an optional step 208, if necessary, selected portions of the implant device 10 can be additionally extended, bent and/or deformed in order to better adapt the implant device 10 to the individual anatomy of the orbital floor of the subject.

Although preferred exemplary embodiments are shown and specified in detail in the drawings and the preceding specification, these should be viewed as purely exemplary and not as limiting the invention. It is noted in this regard that only the preferred exemplary embodiments are shown and specified, and all variations and modifications should be protected that presently or in the future lie within the scope of protection of the invention as defined in the claims.

## Claims

1. An implant device (10) for treating an orbital floor fracture,
wherein the implant device (10) is foldable in a first direction between a folded state and an unfolded state, wherein in the folded state the implant device (10) has a greater curvature in the first direction than in the unfolded state; and
wherein the implant device (10) is plastically extendible in a second direction from an unextended state to an extended state, wherein the second direction is preferably substantially perpendicular to the first direction, wherein in the extended state the implant device (10) has a longer extension in the second direction than in the unextended state;
wherein the implant device (10) is plastically extendible in the second direction to a length (L) in the second direction equal to or greater than an aperture distance of a human maxillary sinus in an anterior-posterior direction.

2. The implant device (10) of claim 1, wherein the implant device (10) is extendible in the second direction to a length in the second direction 5% to 20% greater than an aperture distance of a human maxillary sinus in an anterior-posterior direction, preferably 10% to 15% greater; and/or
wherein the implant device (10) is extendible in the second direction to a length in the second direction of at least 25 mm, in particular to a length in the second direction of 25 mm to 55 mm, preferably of 28 mm to 48 mm.

3. The implant device (10) of any of the preceding claims, wherein the implant device (10) is bendable in the second direction, such that the implant device (10) can simultaneously extend in the second direction and bend in the second direction.

4. The implant device (10) of any of the preceding claims, wherein in the unextended state the implant device (10) has a length in the second direction 10% to 50% smaller than in the unextended state, preferably 20% to 30% smaller.

5. The implant device (10) of any of the preceding claims, wherein the implant device (10) comprises at least one extensible portion (12) and at least one stabilisation portion (14a, 14b), wherein the at least one extensible portion (12) is plastically extendible in the second direction to said length (L) in the second direction equal to or greater than an aperture distance of a human maxillary sinus in an anterior-posterior direction, and wherein the at least one stabilisation portion (14a, 14b) has a reduced extensibility in the second direction compared to the at least one extensible portion (12).

6. The implant device (10) of any of the preceding claims, wherein the implant device (10) comprises a first support edge (16a) and a second support edge (16b) arranged at opposed ends of the implant device (10) in the second direction, wherein the first and second support edges (16a, 16b) are configured for abutting against opposing sidewalls of a cavity, in particular of a human maxillary sinus, such that the implant device (10) is held by tension against said sidewalls, wherein the first and second support edges (16a, 16b) are preferably arranged in a first stabilisation portion (14a) and in a second stabilisation portion (14b) of the at least one stabilisation portion (14a, 14b) of claim 5, respectively.

7. The implant device (10) of any of the preceding claims, wherein the implant device (10) is a mesh-like device comprising at least one first mesh segment (18-1) and at least one second mesh segment (18-2), wherein the at least one first mesh segment (18-1) is foldable in the first direction, and wherein the at least one second mesh segment (18-2) is plastically extendible in the second direction.

8. The implant device (10) of any of the preceding claims, wherein in the unfolded state the implant device (10) has a width (W) in the first direction equal to or smaller than an aperture distance of a human maxillary sinus in a left-right direction; and/or
wherein in the folded state the implant device (10) is wrapped around the second direction with a wrapping radius (R) of 10 mm or less, preferably of 8 mm or less, more preferably of 5 mm or less.

9. The implant device (10) of any of the preceding claims, further comprising a first manipulation eyelet (20a) and a second manipulation eyelet (20b) mutually separated in the second direction, wherein the implant device (10) can be plastically extended in the second direction by mutually separating the first and second manipulation eyelets (20a, 20b), wherein the first manipulation eyelet (20a) and the second manipulation eyelet (20b) are preferably arranged in a first stabilisation portion (14a) and a second stabilisation portion (14b) of the at least one stabilisation portion (14a, 14b) of claim 5, respectively.

10. The implant device (10) of any of the preceding claims, wherein in the unfolded state the implant device (10) of the invention has approximately the shape of a planar parallelogram, preferably with rounded edges and/or rounded corners, wherein the first and second directions may respectively correspond to the width and the length of the parallelogram; and/or
wherein the implant device (10) comprises or is made of a biocompatible material, preferably a metallic material.

11. An implantation tool (50) for implanting an implant device (10) according to any of the preceding claims at an orbital floor fracture, wherein the implantation tool (50) comprises:
a holding part (51) configured for being held and operated by a user; and
an insertion part (53) configured for being inserted into the body of the subject;
wherein the insertion part (53) comprises an insertion tip (70) configured for holding the implant device (10) in the folded state and in the unextended state, wherein the insertion tip (70) comprises:
an unfolding mechanism configured for unfolding the implant device (10) from the folded state to the unfolded state; and
an extending mechanism configured for extending the implant device (10) from the unextended state to the extended state; and
wherein the holding part (51) further comprises a control mechanism (56) configured for controlling the unfolding mechanism and the extending mechanism.

12. The implantation tool of claim 11, wherein the insertion part (53) is detachably connectable with the holding part (51); and/or
wherein the holding part (51) is configured for being hand-held.

13. The implantation tool of any of claims 11 or 12, wherein the insertion part (53) comprises an elongated shaft (62), wherein the shaft (62) extends from a longitudinal end proximal to the holding part (51) to another longitudinal end of the shaft (62) comprising the insertion tip (70) or connected thereto, wherein the elongated shaft (62) preferably has a length of 3 cm to 12 cm, preferably of 4 cm to 8 cm and/or a diameter of 2 mm to 10 mm, preferably of 3 mm to 6 mm; and/or
wherein the insertion tip (70) is configured for holding the implant device (10) angled with respect to a longitudinal direction of the shaft (62), preferably at an angle of 15° to 60°, more preferably of 20° to 45°.

14. The implantation tool of any of claims 11 to 13, wherein the unfolding mechanism comprises one or more flaps (72a, 72b) movable in a direction corresponding to the first direction of the implant device (10), wherein the one or more flaps (72a, 72b) preferably comprise at least two flaps (72a, 72b) arrangeable substantially coplanar for unfolding the implant device (10); and/or
wherein the extending mechanism comprises at least one first fastener (74a) and at least one second fastener (74b) for fastening and extending the implant device (10), wherein the at least one first fastener (74a) and the at least one second fastener (74b) are movable in a direction corresponding to the second direction of the implant device (10), preferably for an extension distance corresponding to a length difference of the implant device (10) in the extended state with respect to the unextended state.

15. An implantation system for treating an orbital floor fracture of a subject comprising:
an implant device (10) according to any of claims 1 to 10, and
an implantation tool (50) according to any of claims 11 to 14 configured for implanting the implant device (10) at said orbital floor fracture.
